# EUROPEAN PATENT APPLICATION

(11) **EP 3 392 616 A1**
(43) Date of publication of application: **24.10.2018**
(21) Application number: 16875623.7
(22) Date of filing: 13.12.2016
(51) Int. Cl.: G01C 21/26, A61B 5/11

(54) **INFORMATION PROCESSING SYSTEM, INFORMATION PROCESSING DEVICE, INFORMATION PROCESSING METHOD, PROGRAM, AND RECORDING MEDIUM**

(30) Priority: 18.12.2015 JP 2015247206; 06.06.2016 JP 2016112425
(71) Applicant: Ricoh Company, Ltd., Tokyo 143-8555 (JP)
(72) Inventor: EBESU, Takafumi, Tokyo 143-8555 (JP); YOSHIZAWA, Fumio, Tokyo 143-8555 (JP); HATA, Daisuke, Tokyo 143-8555 (JP)
(74) Representative: Schwabe - Sandmair - Marx
(86) International application number: PCT/JP2016/087010
(87) International publication number: WO 2017/104646

(57) **Abstract**

An information processing system includes an atmospheric pressure detector configured to detect atmospheric pressure surrounding an information processing apparatus; a determiner configured to determine a movement state of a movable body by using at least a detection result obtained by the atmospheric pressure detector; and a corrector configured to determine the movement state of the movable body by a method different from a method used by the determiner, in response to detecting a predetermined change in the atmospheric pressure detected by the atmospheric pressure detector.

## Description

### [Technical Field]

The present invention relates to an information processing system, an information processing apparatus, an information processing method, a program, and a recording medium.

### [Background Art]

For example, Pedestrian Dead Reckoning (PDR), etc., is known as a technique for estimating a walking state by using an acceleration sensor, an angular velocity sensor, and an atmospheric pressure sensor. For example, Non-Patent Literature 1 proposes, as a method for estimating a walking state, a method of detecting a state of ascending/descending a staircase from a particular angular velocity pattern appearing when ascending a staircase, and a difference value between peak values of acceleration components in the vertical direction and the traveling direction at the time of descending a staircase.

Furthermore, Patent Literature 1 discloses a technique of averaging the atmospheric pressure values with a walking cycle as the unit period, and determining whether the walking of a subject is ascending or descending a staircase according to changes in atmospheric pressure values.

### [Summary of Invention]

### [Technical Problem]

It is an object of the present invention to provide an information processing system, an information processing apparatus, an information processing method, a program, and a recording medium capable of accurately determining a moving state of a movable body.

### [Solution to Problem]

An information processing system includes an atmospheric pressure detector configured to detect atmospheric pressure surrounding an information processing apparatus; a determiner configured to determine a movement state of a movable body by using at least a detection result obtained by the atmospheric pressure detector; and a corrector configured to determine the movement state of the movable body by a method different from a method used by the determiner, in response to detecting a predetermined change in the atmospheric pressure detected by the atmospheric pressure detector.

### [Advantageous Effects of Invention]

According to the disclosed technique, it is possible to provide an information processing system, an information processing apparatus, an information processing method, a program, and a recording medium capable of accurately determining a moving state of a movable body.

### [Brief Description of Drawings]

FIG. 1 is a block diagram illustrating an example of hardware of an information processing apparatus used in a navigation system according to a first embodiment;
FIG. 2 is a block diagram illustrating an example of functions of software in the information processing apparatus according to the first embodiment;
FIG. 3 is a flowchart (part 1) of an example of a walking state determination method executed by the information processing apparatus according to the first embodiment;
FIG. 4 is a diagram illustrating a contrast of acceleration changes and stride lengths during walking;
FIG. 5 is a flowchart (part 2) of an example of a walking state determination method executed by the information processing apparatus according to the first embodiment;
FIG. 6 illustrates an example of walking history information according to the first embodiment;
FIG. 7 is a graph illustrating atmospheric pressure variations during normal walking, with the horizontal axis representing the walking time (sec) and the vertical axis representing the atmospheric pressure (hPa) acquired by the atmospheric pressure sensor 114 when the user is performing normal walking;
FIG. 8 is a graph illustrating atmospheric pressure variations detected by the atmospheric pressure sensor 114 when a train passes, with the horizontal axis representing the walking time (sec) and the vertical axis representing the atmospheric pressure (hPa) acquired by the atmospheric pressure sensor 114, similar to FIG. 7;
FIG. 9 illustrates atmospheric pressure variations at the time of normal walking and atmospheric pressure variations when a train passes a platform indicated in a single graph for the purpose of comparison;
FIG. 10A is a diagram (before correction) for describing an example of correcting the walking state by using walking history information according to the first embodiment;
FIG. 10B is a diagram (after correction) for describing an example of correcting the walking state by using walking history information according to the first embodiment;
FIG. 11 is a block diagram illustrating an example of functions of a navigation system 1100 according to a second embodiment;
FIG. 12 is a block diagram illustrating an example of functions of a navigation system 1200 according to a third embodiment;
FIG. 13 is a block diagram illustrating an example of functions of a navigation system 1300 according to a fourth embodiment;
FIG. 14 is a flowchart (part 1) of an example of a walking state determination method in a navigation system according to the fourth embodiment;
FIG. 15 is a flowchart (part 2) of an example of a walking state determination method in a navigation system according to the fourth embodiment;
FIG. 16A is a diagram (before correction) for describing an example of correcting the walking state by using walking history information according to the fourth embodiment; and
FIG. 16B is a diagram (after correction) for describing an example of correcting the walking state by using walking history information according to the fourth embodiment.

### [Description of Embodiments]

According to the conventional method, there have been cases where the accuracy deteriorates due to the fact that the portable device is fixed to the waist portion, or due to the way of walking by the individual, etc.

Furthermore, for example, in the technique disclosed in Patent Literature 1, there may be a case where the walking state cannot be determined correctly, when a particular atmospheric pressure change occurs due to a motion other than vertical movements during the walking motion, etc.

In the following, the present invention will be described with reference to embodiments; however, the present invention is not limited to the embodiments described below.

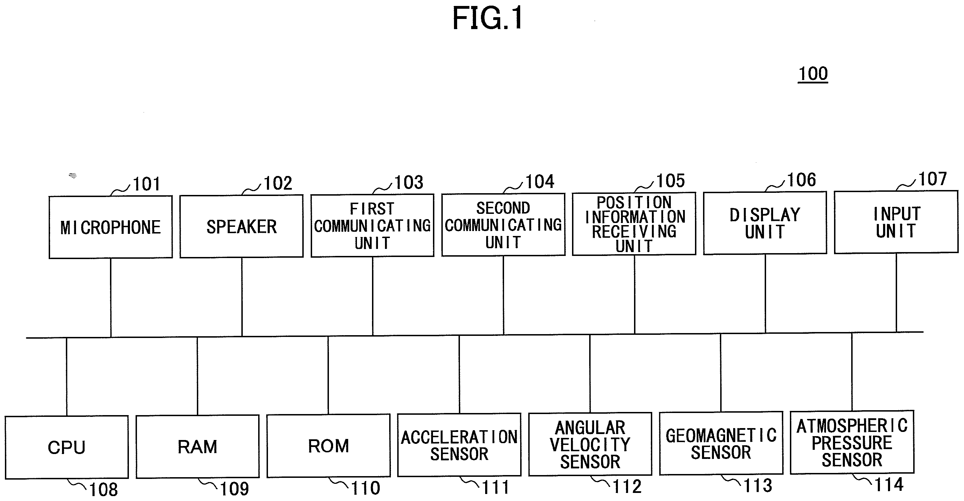

FIG. 1 is a block diagram illustrating an example of hardware of an information processing apparatus 100 used in a navigation system according to a first embodiment. Note that the navigation system according to the first embodiment in which the information processing apparatus 100 is used, is an example of an information processing system.

The information processing apparatus 100 is a movable body such as a mobile phone, a smartphone, a Personal Digital Assistant (PDA), or a notebook personal computer, etc., and is a device that can be carried by a user in his/her hand.

The block diagram illustrated in FIG. 1 is a block diagram of a case where the information processing apparatus 100 is a portable terminal such as a smartphone. The information processing apparatus 100 includes a Central Processing Unit (CPU) 108, a Random Access Memory (RAM) 109, and a Read-Only Memory (ROM) 110. The CPU 108 executes programs for controlling the operations of the information processing apparatus 100, and the RAM 109 provides a work area of the CPU 108, etc. The ROM 110 stores programs executed by the CPU 108 and data necessary for executing the programs.

Furthermore, the information processing apparatus 100 may include an acceleration sensor 111, an angular velocity sensor 112, a geomagnetic sensor 113, and an atmospheric pressure sensor 114 that is an example of an atmospheric pressure detecting unit. The acceleration sensor 111 is an example of an acceleration detecting unit that detects the acceleration, for example, in three axial directions. The angular velocity sensor 112 is an example of an angular velocity detecting unit, and detects the angular velocity, for example, in three axial directions. The geomagnetic sensor 113 is an example of a magnetic detector, and outputs a three-dimensional vector representing magnetic north, and is used for detecting the orientation of the information processing apparatus 100. The acceleration sensor 111, the angular velocity sensor 112, and the geomagnetic sensor 113 are used as motion sensors, and are used for detecting the walking speed, the number of steps, and the traveling direction of the user. The acceleration sensor 111, the angular velocity sensor 112, and the geomagnetic sensor 113 provide inertial information.

The atmospheric pressure sensor 114 detects the atmospheric pressure around the user, that is, around the information processing apparatus 100 carried by the user. The information processing apparatus 100 detects the change in the position of the user in the altitude direction based on the information obtained by the atmospheric pressure sensor 114, and adds the information, which is obtained by the atmospheric pressure sensor 114, to two-dimensional position coordinate information of the user acquired by a motion sensor, as altitude information indicating the altitude. As a result, the information processing apparatus 100 can determine the position of the user within the three-dimensional position coordinates.

In addition, the information processing apparatus 100 includes a microphone 101, a speaker 102, a first communicating unit 103, and a second communicating unit 104. The microphone 101 converts a sound such as a voice of a user into electric signals, enabling telephone communication, etc. The speaker 102 generates a sound of an incoming call of telephone communication, etc., and outputs sound signals converted from electric signals received by a telephone communication line. Furthermore, the first communicating unit 103 performs communication processing for connecting the information processing apparatus 100 to a wide area network such as Wifi, 3G, 4G, or Long Term Evolution (LTE), etc. The second communicating unit 104 performs processing for providing short-range communication such as Bluetooth (registered trademark), Infrared Data Association (IRDA), or short-range wireless communication, etc.

Furthermore, the information processing apparatus 100 includes a position information receiving unit 105, a display unit 106, and an input unit 107. The information processing apparatus 100 receives, by the position information receiving unit 105, positioning signals transmitted by a global positioning system (GPS) satellite or an Indoor Messaging Service (IMES), and acquires the position coordinates of the user. Furthermore, the display unit 106 and the input unit 107 provide a touch panel function, for example, using a liquid crystal display, etc., and provide a user interface between the information processing apparatus 100 and the user. Note that the microphone 101, the speaker 102, the first communicating unit 103, the second communicating unit 104, the position information receiving unit 105, the display unit 106, and the input unit 107 are optional elements, and the information processing apparatus 100 does not need to include all of the hardware elements illustrated in FIG. 1.

Furthermore, the information processing apparatus 100 may include a memory card interface, a Universal Serial Bus (USB) interface, and an imaging device, etc. Specific examples of the CPU 108 used by the information processing apparatus 100 are PENTIUM (registered trademark) to PENTIUM IV (registered trademark), PENTIUM (registered trademark) compatible CPU, POWER PC (registered trademark), MIPS, Tegra (registered trademark), Snapdragon (registered trademark), and Helio (registered trademark), etc., according to the implementation of the information processing apparatus 100.

Furthermore, as the operating system (OS) used by the information processing apparatus 100, there are MacOS (trademark), i-OS (registered trademark), Windows (registered trademark), CHROME (registered trademark), ANDROID (registered trademark), Windows (registered trademark) 200X Server, UNIX (registered trademark), AIX (registered trademark), LINUX (registered trademark), or any other suitable OS, according to the implementation of the information processing apparatus 100. Furthermore, the information processing apparatus 100 can store and execute application programs, which operate on the aforementioned OS, described in a programming language such as C, C ++, Visual C++, Visual Basic, Java (registered trademark), Perl, and Ruby, etc., and a programming language often used in smartphones.

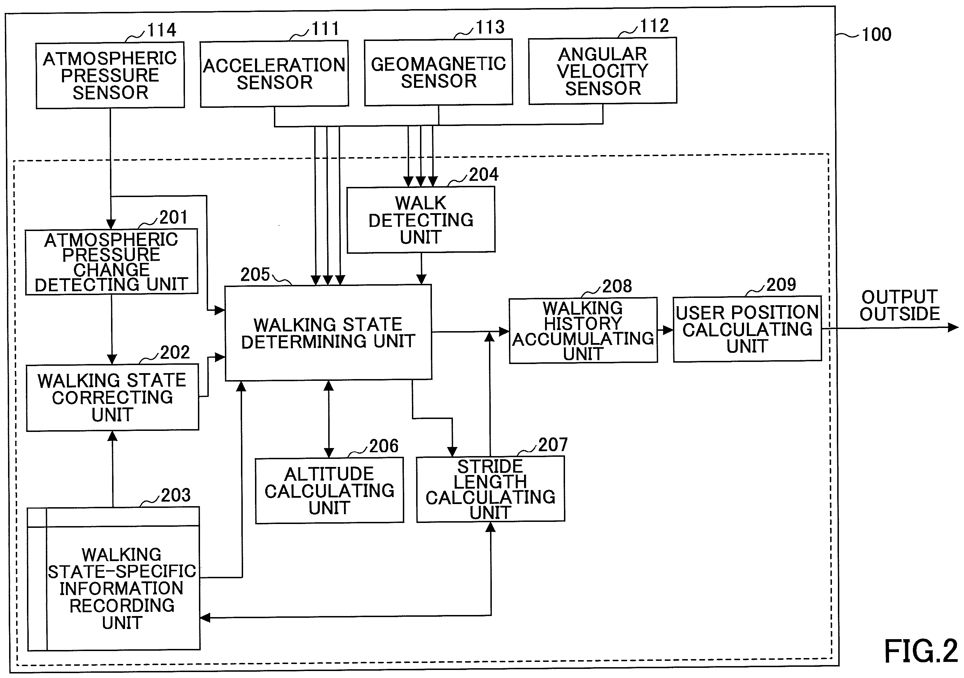

FIG. 2 is a block diagram illustrating an example of functions of software in the information processing apparatus 100 according to the first embodiment. Note that in FIG. 2, the elements surrounded by broken lines are examples of functions implemented as the CPU 108 executes programs. As a matter of convenience, the atmospheric pressure sensor 114, the acceleration sensor 111, the geomagnetic sensor 113, and the angular velocity sensor 112 are also illustrated in FIG. 2; however, these sensors are illustrated for describing the data flows of the respective signals, and are not included among the software functions.

The software functions in the information processing apparatus 100 include an atmospheric pressure change detecting unit 201, a walk detecting unit 204, and a walking state determining unit 205. The atmospheric pressure change detecting unit 201 acquires the atmospheric pressure information acquired by the atmospheric pressure sensor 114, and detects a change with time in the atmospheric pressure information (that is, a change with time in the atmospheric pressure). The atmospheric pressure change detecting unit 201 detects, for example, a rapid atmospheric pressure change accompanying the passage of a train in a station, from a value indicating a predetermined atmospheric pressure change or an atmospheric pressure variance value within a predetermined time range.

The walk detecting unit 204 detects the walking of the user by using the information acquired by the acceleration sensor 111, the geomagnetic sensor 113, and the angular velocity sensor 112. The walking state determining unit 205 also uses the information acquired from the acceleration sensor 111, the geomagnetic sensor 113, and the angular velocity sensor 112, to acquire walking history information 600 indicating the walking history of the walking of the user. More specifically, the walking state determining unit 205 adds information of the height dimension to the information indicating the user's walking in a horizontal plane, and generates the user's walking history information 600, which is an example of the movement history information, as three-dimensional information. Furthermore, the walking state determining unit 205 determines which state the user's walking state is in, among a flat walking state (that is, walking on a substantially horizontal surface), and a state of ascending/descending a staircase, etc., based on the atmospheric pressure information acquired by the atmospheric pressure sensor 114, by referring to a walking state-specific information recording unit 203.

Furthermore, the information processing apparatus 100 may include a walking state correcting unit 202, an altitude calculating unit 206, a stride length calculating unit 207, and a walking history accumulating unit 208. The walking history accumulating unit 208 stores the walking history information 600 of a user generated by the walking state determining unit 205. The altitude calculating unit 206 receives the atmospheric pressure information acquired by the atmospheric pressure sensor 114 via the walking state determining unit 205, calculates the altitude information, and returns the altitude information to the walking state determining unit 205. The walking state determining unit 205 determines the walking state by using altitude information from the altitude calculating unit 206.

The stride length calculating unit 207 refers to the walking state-specific information recording unit 203, calculates the stride length of the user in the walking state of the user determined by the walking state determining unit 205 (or further determined by the walking state correcting unit 202), and reports the calculated stride length to the walking history accumulating unit 208. As described above, the stride length calculating unit 207 updates the stride length information indicating the stride length that changes depending on the walking state, in the walking history information 600 stored in the walking history accumulating unit 208. The walking history accumulating unit 208 stores the walking state of the user in time series as a walking log, based on the information acquired by each of the above sensors. Furthermore, the information processing apparatus 100 may include a user position calculating unit 209.

The user position calculating unit 209 uses the walking history information 600 stored in the walking history accumulating unit 208 to determine the position by Pedestrian Dead Reckoning, for example, and the user position calculating unit 209 three-dimensionally calculates the position of the user starting from the position where the user's walking has been first detected, to make it possible to display the walking route of the user, for example, by superimposing the walking route on a map. For example, the user's position where the user's walking is first detected, can be detected by receiving positioning signals transmitted by a GPS satellite or the IMES by the position information receiving unit 105 to acquire the position coordinates of the user.

Note that in the walking state-specific information recording unit 203, parameters of the user's walking, such as information indicating the altitude change in the case of flat walking, information indicating the altitude change when ascending/descending a staircase, and information indicating the altitude change in various walking statuses such as a running state, are recorded. In the walking state-specific information recording unit 203, level difference information indicating the difference in the levels of stairs when ascending/descending a staircase is recorded in order to calculate the stride length, etc. Note that the level difference information may be set in advance based on a value of a general staircase (for example, a value of a standard of staircases).

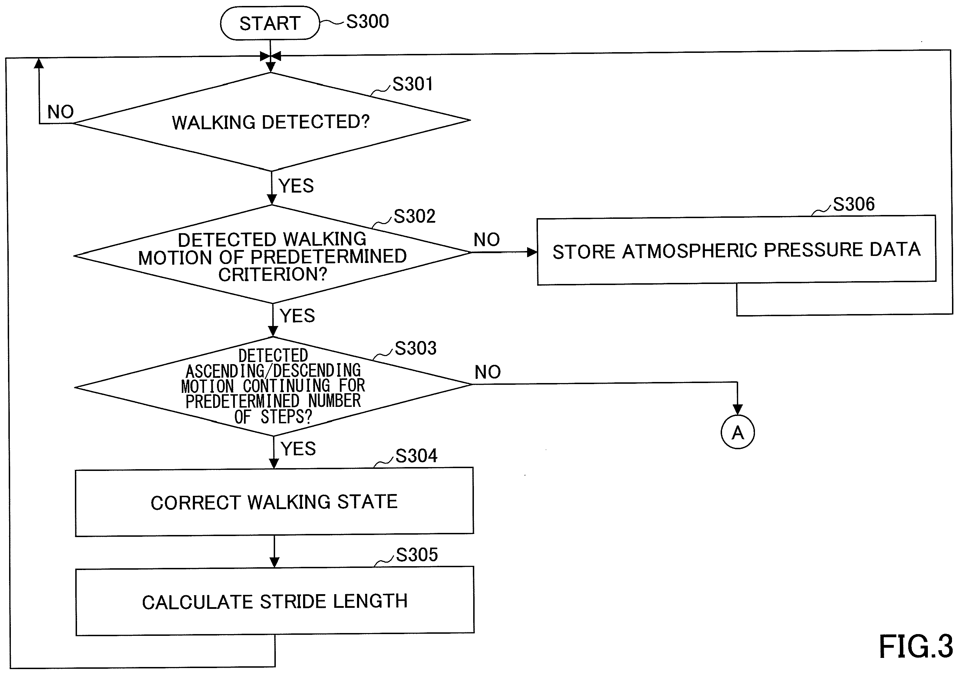

FIG. 3 is a flowchart of an example of a walking state determination method that is an example of an information processing method executed by the information processing apparatus 100.

When the process of FIG. 3 is started at step S300, for example, by an operation by a user, in step S301, the walk detecting unit 204 detects the walking of the user by using acceleration information indicating the acceleration detected by the acceleration sensor 111. In step S302, the walk detecting unit 204 determines whether the detected walking has continued for a predetermined criterion. When the walking state determining unit 205 determines that the detected walking has not continued for the predetermined criterion (NO), in order to acquire an initial value of the atmospheric pressure information, in step S306, the walking state determining unit 205 stores the atmospheric pressure information for the period during which the walking has continued, and returns to step S301.

Conversely, in step S302, when the walking state determining unit 205 determines that the detected walking has continued for the predetermined criterion (YES), the walking state determining unit 205 proceeds to step S303. Note that the predetermined criterion for determining whether the walking has continued, can be set by using the walking time, or the number of steps, etc. For example, it can be determined that the detected walking has continued for the predetermined criterion, when the detected walking has continued for five steps when the criterion is the number of steps, or when the detected walking has continued for 2.5 seconds when the criterion is the walking time. The predetermined criterion for determining whether the walking has continued, can be appropriately set according to the walking characteristics, etc., of the user.

In step S303, the walking state determining unit 205 determines whether the ascending/descending state of the user has continued for a "predetermined number of steps". When the ascending/descending motion of the user continuing for the predetermined number of steps has been detected (YES), the walking state determining unit 205 proceeds to step S304. In step S304, the walking state correcting unit 202 corrects the determination of the walking state to "flat walking", and records "flat walking" in the walking history information 600 stored in walking history accumulating unit 208. Then, in step S305, according to the walking state of the "flat walking", the stride length calculating unit 207 calculates the "stride length", and records the calculated stride length in the walking history information 600.

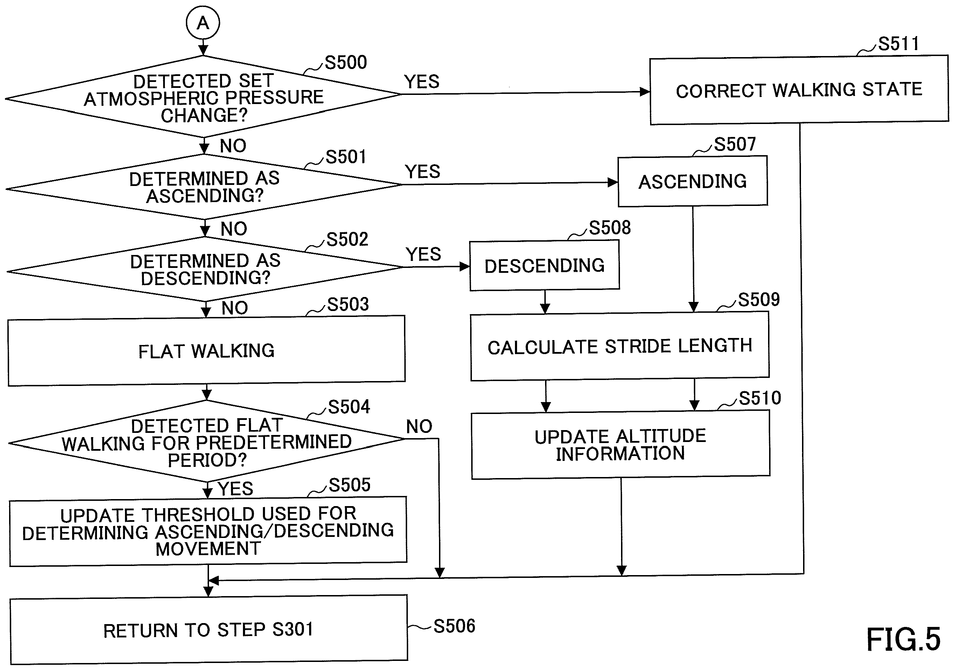

Here, in step S303, when it is determined that a state of "ascending" or "descending", as the determination result of the walking state in steps S500 to S503, S507, S508, and S511 in FIG. 5 described later, has continued for a "predetermined number of steps", the walking state correcting unit 202 determines that the determination of the predetermined step number is an error, and corrects the determination to "flat walking" in step S304. According to standards, etc., of buildings, usually, the stairway in a building is provided with a landing area at every predetermined number of stairs. Therefore, when the "ascending" or "descending" determination continues beyond a predetermined number of stairs, the final determination is determined to be an error, and the determination is corrected to "flat walking". That is, a value obtained by adding "1" to the predetermined number of stairs is the value of the "predetermined number of steps".

Conversely, when it is determined in step S303 that the ascending/descending state continuing for the predetermined number of steps has not been detected (that is, "ascending" or "descending" is not continued for a predetermined number of steps) (NO), the processes from step S500 and onward in FIG. 5 are executed. The flowchart of FIG. 5 will be described later.

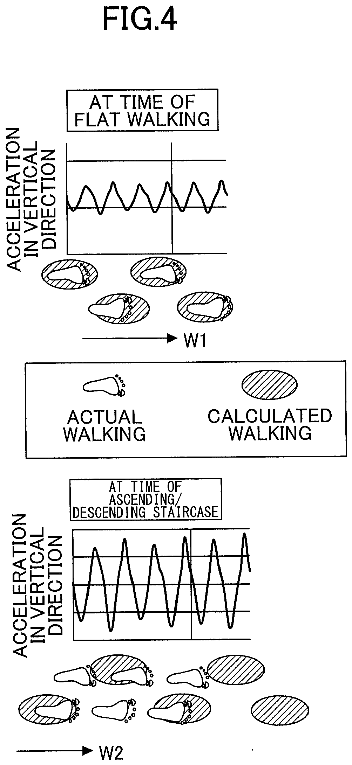

Next, the calculation of the stride length will be described with reference to FIG. 4. FIG. 4 illustrates a contrast of examples of acceleration changes in the vertical direction (graph) and stride lengths during walking, between a case of flat walking and a case of ascending/descending a staircase.

As illustrated in the upper part of FIG. 4, at the time of flat walking, the acceleration change in the vertical direction detected by the acceleration sensor 111 is relatively small, and a stride length W1 is wider than a stride length W2 in the case of ascending/descending a staircase as illustrated in the lower part of FIG. 4. Conversely, as illustrated in the lower part of FIG. 4, at the time of ascending/descending a staircase, the stride length is W2 that is substantially equal to the width of each of the stairs of the staircase, whereas the acceleration in the vertical direction detected by the acceleration sensor 111 varies within a relatively large range. Note that the walking speed is acquired by calculating the moving speed in the horizontal direction from the amplitude of the acceleration in the vertical direction, for example. In this case, as illustrated in FIG. 4, the amplitude of the acceleration in the vertical direction at the time of ascending/descending a staircase, is larger than that at the time of flat walking, and therefore the walking speed at the time of ascending/descending a staircase acquired by the above calculation tends to be higher than the actual walking speed. Furthermore, in the case of ascending/descending a staircase, the stride length is actually limited by the tread of the stairs, so the above tendency is considered to be more prominent.

Flat walking and ascending/descending a staircase can be distinguished by using the variance value of the acceleration in the vertical direction and/or the atmospheric pressure information acquired by the atmospheric pressure sensor 114. As described above, the stride length W2 at the time of ascending/descending a staircase is narrower than the stride length W1 at the time of flat walking, and therefore when the moving distance of the user is calculated by multiplying by the number of steps with reference to the stride length at the time of flat walking, an error may occur in detecting the present position. In order to avoid such a situation, for example, when it is determined that the walking of the user is ascending/descending a staircase, the information processing apparatus 100 corrects the stride length from the value at the time of flat walking to the stride length at the time of the ascending/descending motion. Furthermore, in this case, when the walking of the user changes from ascending/descending a staircase to flat walking, the information processing apparatus 100 corrects the stride length at the time of ascending/descending a staircase back to the stride length at the time of the flat walking.

Note that when GPS, etc., can be used, the information processing apparatus 100 can correct the present position with the information obtained by GPS, etc.; however, in an underground structure such as the subway, or inside a building, etc., GPS may not function properly. In such a case, it is effective to correct the stride length as described above (that is, by using different methods of calculating the stride length between the case of flat walking and the case of ascending/descending walking), in order to reduce the detection error of the present position.

In the following, description of the walking state determination method will be continued by using FIG. 5. The process in FIG. 5 is started when the result of the determination in step S303 in FIG. 3 is NO. First, in step S500, it is determined whether the atmospheric pressure change detecting unit 201 has detected a "preset atmospheric pressure change".

The above-described "preset atmospheric pressure change" means, for example, a rapid change in atmospheric pressure that does not occur during regular walking, such as a pressure change when a train arrives or departs in a subway station (hereinafter referred to as "rapid atmospheric pressure change"). Information indicating such a "rapid atmospheric pressure change" may be recorded as data in the ROM 110 of the information processing apparatus 100, for example. Furthermore, the presence or absence of the "rapid atmospheric pressure change" may be determined, for example, by determining whether the atmospheric pressure difference between the present atmospheric pressure and the atmospheric pressure before the user walks a predetermined number of steps exceeds a predetermined threshold, or by determining whether a variance value of the atmospheric pressure within a predetermined time range exceeds a predetermined threshold. Note that the determination criterion for determining the presence or absence of the "rapid atmospheric pressure change" may be set to any criterion.

When a "rapid atmospheric pressure change" ("the set atmospheric pressure change" in FIG. 5) is not detected (NO in step S500), the walking state determining unit 205 executes step S501 to determine whether the user's walking is an ascending motion, and when it is determined that the walking is an ascending motion (YES), the walking state determining unit 205 proceeds to step S507. In step S507, the walking state determining unit 205 records the walking state of the user as an ascending motion (for example, "ascending staircase") in the walking history information 600.

Next, in step S509, the stride length calculating unit 207 calculates the stride length by the method described above with reference to FIG. 4. Subsequently, in step S510, the walking state determining unit 205 calculates the altitude information by using the information that indicates the level difference of stairs at the time of ascending/descending and that is stored in the walking state-specific information recording unit 203, and uses the calculated altitude information to update the walking history information 600 stored in the walking history accumulating unit 208. Note that the information that indicates the level difference of stairs at the time of ascending/descending may be set in advance based on a value of a general staircase (the value of a standard of staircases, etc.). Subsequently, step S506 is executed, and step S301 of FIG. 3 is further executed, whereby the process described above with reference to FIG. 3 is repeated.

In the calculation of the stride length, for example, in the calculation of the walking speed at the time of ascending/descending, the stride length calculating unit 207 multiplies the walking speed, which is calculated by using the acceleration in the vertical direction, by a coefficient considering the inclination and the level difference of stairs, etc., to calculate the walking speed in the horizontal direction at the time of ascending/descending, and further calculates the stride length by using the calculated walking speed and the number of steps. Note that the coefficient considering the inclination and the level difference of stairs, etc., may be obtained in advance based on a value of a general staircase or a slope (for example, a value of a standard of staircases), etc., and recorded in the walking state-specific information recording unit 203. Then, by reporting the stride length calculated by the stride length calculating unit 207 to the walking state determining unit 205, the walking state determining unit 205 records the stride length in the walking history information 600 stored in the walking history accumulating unit 208.

Conversely, in step S501, when it is not determined that the user's walking state is an ascending motion (NO), the walking state determining unit 205 proceeds to step S502 to determine whether the user's walking state is a descending motion. In step S502, when it is determined that the walking state of the user is a descending motion (YES), the walking state determining unit 205 proceeds to step S508 and records the walking state of the user as a descending motion (for example, "descending staircase") in the walking history information 600.

Next, in step S509, the stride length calculating unit 207 calculates the stride length by the method described above with reference to FIG. 4. Subsequently, in step S510, the walking state determining unit 205 calculates the altitude information by using the information indicating the level difference of stairs at the time of ascending/descending that is stored in the walking state-specific information recording unit 203, and uses the calculated altitude information to update the walking history information 600 stored in the walking history accumulating unit 208. Subsequently, step S506 is executed, and step S301 of FIG. 3 is further executed, whereby the process described above with reference to FIG. 3 is repeated.

Conversely, in step S502, when it is not determined that the walking state of the user is a descending motion (NO), the walking state determining unit 205 proceeds to step S503, determines the walking state of the user as "flat walking" and records "flat walking" in the walking history information 600, and the stride length calculating unit 207 calculates the number of steps according to "flat walking" and records the number of steps in the walking history information 600.

Furthermore, the walking state determining unit 205 proceeds to step S504, and determines whether flat walking has been continuously detected for a predetermined time. When flat walking is not continuously detected for a predetermined time (NO in step S504), step S506 is executed, and step S301 in FIG. 3 is further executed, whereby the process described above with reference to FIG. 3 is repeated.

In step S504, when flat walking is continuously detected for a predetermined time (YES), the walking state determining unit 205 proceeds to step S505. In step S505, the walking state determining unit 205 updates a threshold that is to be referred to when determining whether the user's walking state is ascending/descending a staircase, etc., and that is stored in the walking state-specific information recording unit 203, by using, for example, a variance value of the acceleration in the vertical direction during the flat walking continuously detected. As a result, it is possible to improve the accuracy in determining whether the walking state is ascending/descending a staircase, etc. Subsequently, step S506 is executed, and step S301 of FIG. 3 is further executed, whereby the process described above with reference to FIG. 3 is repeated.

Conversely, in step S500, when "rapid atmospheric pressure change" is detected (YES), the walking state correcting unit 202 determines that the walking state will be erroneously determined due to a factor other than the user's movement such as ascending/descending a staircase (for example, a rapid atmospheric pressure change accompanying the passage of a train in a station). That is, the walking state correcting unit 202 determines that the user's walking state cannot be determined by the original determination method based on the atmospheric pressure information from the atmospheric pressure sensor 114. Therefore, the walking state correcting unit 202 proceeds to step S511 to acquire the "walking state" to be recorded in the walking history information 600, by a method other than the original determination method. Acquisition of a walking state by a method other than the original determination method means, for example, that the walking state correcting unit 202 uses the "walking state" determined before the "rapid atmospheric pressure change" is detected, as the "walking state" to be recorded in the walking history information 600.

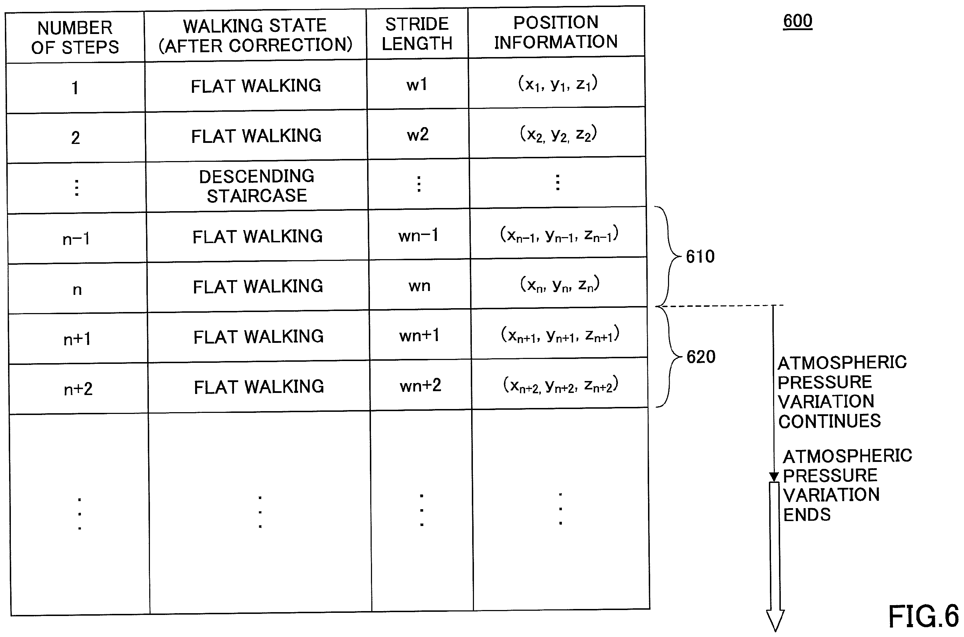

In the following, with reference to FIG. 6, the process of determining the walking state performed by the walking state correcting unit 202, with respect to the walking state to be recorded in the walking history information 600 stored in the walking history accumulating unit 208, will be described in more detail. FIG. 6 illustrates an example of the walking history information 600. The walking history information 600 illustrated in FIG. 6 starts to be recorded when the user starts recording the walking state using the information processing apparatus 100, and includes the number of steps (cumulative), the walking state, the stride length, and the position information, etc. Note that when the information acquired by GPS, etc., can be used, a value acquired by GPS, etc., may be used as the position information. Furthermore, when GPS, etc., cannot be used indoors or in an underground space, etc., it is possible to use position information that is calculated by using inertial information such as the number of steps, the stride length, the acceleration, and the angular velocity, etc., and atmospheric pressure information, etc.

In the walking history information 600 illustrated in FIG. 6, it is assumed that a pressure change greater or equal to a preset threshold (that is, a "rapid atmospheric pressure change") is detected at the time point when an n number of steps n is detected. In such a case, if the walking state determining unit 205 determines that the walking state of the user is an "ascending/descending motion" (that is, "ascending" or "descending") according to the above-described "rapid atmospheric pressure change", this kind of determination affects the walking state and stride length to be recorded in the walking history information 600. For example, when such a situation continues for several seconds, it is conceivable that a non-negligible error in detecting the position information of the user, will occur. For this reason, for example, when the atmospheric pressure changes beyond a preset threshold value as compared with the atmospheric pressure before a predetermined number of steps, the atmospheric pressure change detecting unit 201 determines that this atmospheric pressure change is a "rapid atmospheric pressure change", and determines that the atmospheric pressure change is not caused by walking but is caused by external factors (for example, a rapid atmospheric pressure change accompanying the passage of a train in a station).

When such a determination is made, the walking state correcting unit 202 determines the "walking state" to be recorded in the walking history information 600, by a method different from the original determination method. The "walking state" to be acquired by a determination by a method different from the original determination method, is the walking state of a predetermined number of steps; in the embodiment illustrated in FIG. 6, the walking state of two steps. That is, as illustrated in FIG. 6, the walking state correcting unit 202 uses the value of a walking state 610 before the "rapid atmospheric pressure change" is detected, as a walking state 620 after the atmospheric pressure change is detected. In the example of FIG. 6, the walking state correcting unit 202 corrects the determination of the walking state of "ascending staircase", which is determined by the original determination method (that is, the determination performed by the walking state determining unit 205 based on the atmospheric pressure information from the atmospheric pressure sensor 114), to "flat walking". Note that "stride length" is calculated in real time from w1 to wn+2 by the stride length calculating unit 207 by using "walking state" determined by the walking state determining unit 205 (or determined by the walking state correcting unit 202), and records the calculated stride length in the walking history information 600.

Furthermore, in the walking history information 600, for example, when "rapid change in atmospheric pressure" is detected at the nth step, the "walking state" at the nth step is also used as the "walking state" at the (n+1)th step, and furthermore, during the time indicated by the arrow line of " atmospheric pressure variation continues", that is, while the atmospheric pressure that has changed due to the above atmospheric pressure change continues, the "walking state" of the nth step may also be repeatedly used as the subsequent "walking states".

The atmospheric pressure change detecting unit 201 can determine that the continuation of the atmospheric pressure, which has changed by the rapid atmospheric pressure change, has ended, for example, by detecting that the atmospheric pressure has returned to the level of the average atmospheric pressure before the rapid atmospheric pressure change is detected. At this time, for example, when a rapid atmospheric pressure change occurs, the "walking state" determined by the original determination method may be temporarily recorded as the subsequent "walking state" in the walking history information 600, instead of recording, in real time, the "walking state" using the past data or the latest data, as the subsequent "walking state" in the walking history information 600. In this case, for example, assuming that the atmospheric pressure gradually decreases from the atmospheric pressure, which has changed by the rapid atmospheric pressure change, and reaches the level of the average atmospheric pressure, and then the atmospheric pressure further decreases, the "number of steps", which is detected at the time point when the atmospheric pressure crosses the average atmospheric pressure level, is recorded, and the "walking state" in the walking history information 600 is corrected retroactively up to the recorded number of steps by using the past data or the latest data.

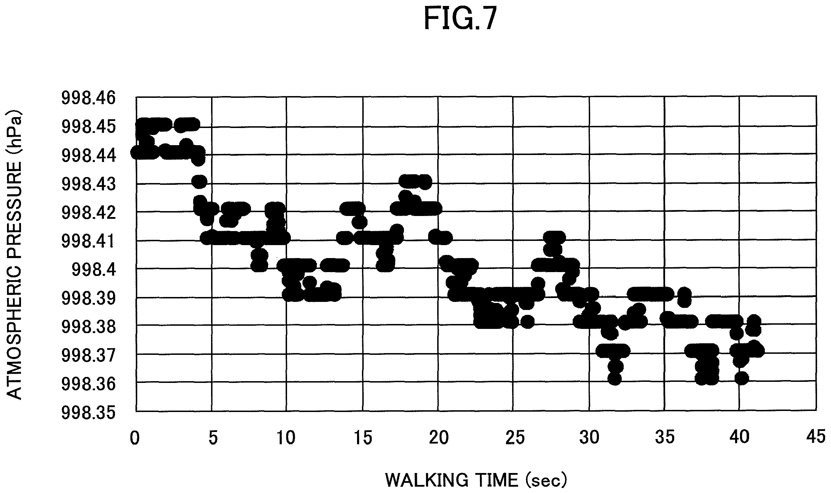

FIG. 7 illustrates an example of atmospheric pressure variations when the user is performing normal walking, with the horizontal axis representing the walking time (sec) and the vertical axis representing the atmospheric pressure (hPa) acquired by the atmospheric pressure sensor 114. As illustrated in FIG. 7, while the user is walking normally, there are pressure variations; however, the pressure variations fall within a range of approximately 1.2×10⁻² hPa/sec or less.

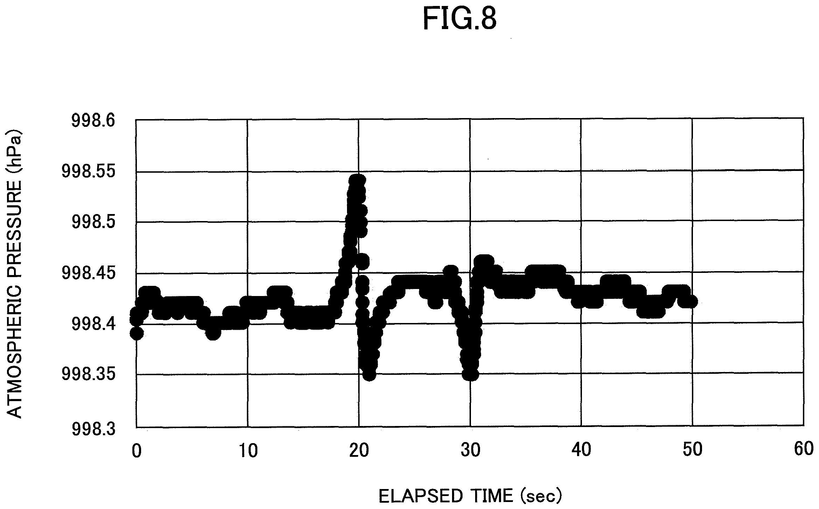

FIG. 8 illustrates an example of atmospheric pressure variations detected by the atmospheric pressure sensor 114 when a train passes, with the horizontal axis representing the walking time (sec) and the vertical axis representing the atmospheric pressure (hPa) acquired by the atmospheric pressure sensor 114, similar to FIG. 7. As illustrated in FIG. 8, for example, when a train, etc., enters the platform of a train station, a pressure variation in a range of approximately 7.5×10⁻² hPa/sec or more occurs.

These pressure variation values can be used as thresholds for determining whether there is a "rapid atmospheric pressure change". However, the atmospheric pressure variations illustrated in FIGS. 7 and 8 are merely examples, and the atmospheric pressure varies depending on the location and the environment, etc., where the user is present, and a specific threshold may be set appropriately.

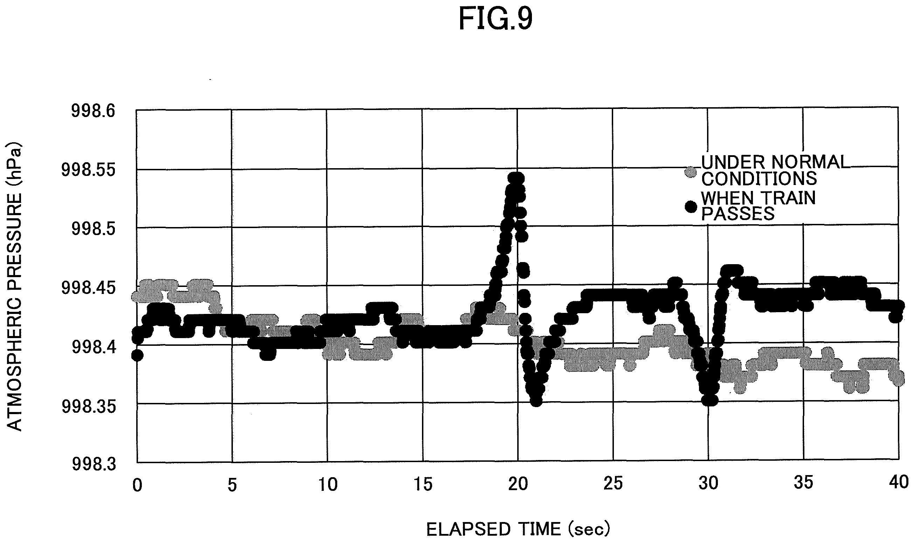

FIG. 9 illustrates the pieces of atmospheric pressure variation data illustrated in FIGS. 7 and 8 in an overlapping manner in a single graph for the purpose of comparing the pressure variation during normal walking and the pressure variation when a train passes a platform. As illustrated in FIG. 9, when the atmospheric pressure varies due to external factors such as the atmospheric pressure variation when a train passes a platform, the atmospheric pressure changes much more abruptly than at the time of normal walking, and the atmospheric pressure after the change continues for a relatively long time. From the above, for example, it is possible to set an atmospheric pressure change, for which the absolute value of the atmospheric pressure variation speed is greater than or equal to a predetermined value, as the "rapid atmospheric pressure change".

Besides the above, for example, as a threshold for determining whether there is a "rapid atmospheric pressure change", different values may be set for different cases, such as a case where the user is in an elevator and a case where the user is on an escalator, etc., and the determination of the walking state and the calculation of the stride length may be performed in a mode according to each case. This is because, for example, in a case where a user is in an elevator, or when the user is on an escalator, even if a walking motion is detected, the stride length may be different from that during normal walking, or it may not be appropriate to recognize the detected walking motion as a change in the position.

Furthermore, as described above, even if a rapid atmospheric pressure change occurs, instead of recording, in real time, the "walking state" using the past data or the latest data, it is possible to continue recording the "walking state" determined by the original determination method (using the atmospheric pressure information). In this case, the duration of the atmospheric pressure, which has changed by the rapid atmospheric pressure change, is measured, and subsequently, the "walking state" corresponding to the duration in the walking history information 600 is corrected retroactively to when the rapid atmospheric pressure change has started, to the "walking state" defined by using the past data or the latest data.

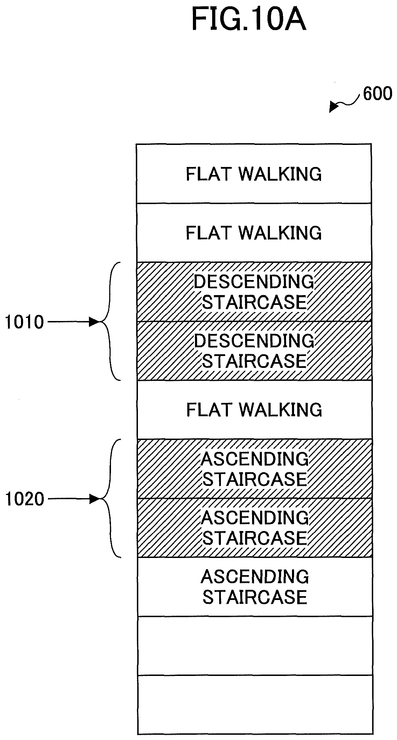

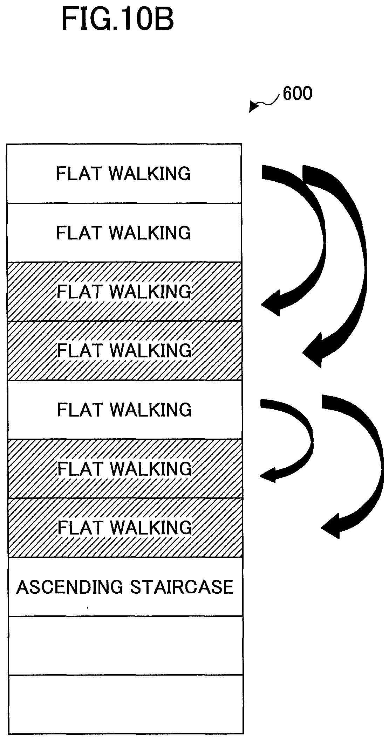

FIGS. 10A and 10B are diagrams for describing an example of acquiring the "walking state" in the walking history information 600. In FIGS. 10A and 10B, a walking state 1010 is a walking state determined when a train enters a platform, and a walking state 1020 is a walking state determined when the train passes the platform. During these walking states, the atmospheric pressure change detecting unit 201 detects an atmospheric pressure variation speed larger than a threshold (that is, a "rapid atmospheric pressure change"), and therefore, the walking state correcting unit 202 uses the past data or the latest data as the "walking state" in the walking history information 600, for a duration of the atmospheric pressure following the rapid atmospheric pressure change. Accordingly, the stride length calculating unit 207 calculates the "stride length" in the walking history information 600 according to the "walking state" based on the past data or the latest data.

As the "stride length", the stride lengths such as W1 and W2 described with reference to FIG. 4 can be calculated, in real time, by using the walking speed and the number of steps obtained by using a motion sensor. Furthermore, as the "walking state", when there is no large difference in the detected value of the acceleration detected by the acceleration sensor 111, it can be determined that the walking state of the user, before the occurrence of the "rapid atmospheric pressure change", is maintained.

For example, it is possible to acquire the walking state by a method different from the original determination method, by copying and using data of an appropriate number of steps before a predetermined number of steps, as the data to be recorded in the walking history information 600, and it is possible to calculate the stride length according to the obtained walking state. For example, in FIG. 10A and FIG. 10B, the walking state 1010 of two steps is obtained by copying and using the walking state of two steps before and the walking state of three steps before, respectively. Furthermore, the walking state 1020 of two steps is obtained by copying and using the walking state of one step before and the walking state of two steps before, respectively. In this case, the walking state of how many steps before to be actually copied and used may be appropriately determined according to the occurrence status of the walking state to be corrected. Note that when the acceleration value of the acceleration sensor 111 varies at the same time as when a rapid atmospheric pressure change occurs, the walking state obtained from the acceleration sensor 111, for example, a walking state corresponding to a case where the user starts running, may be recorded in the walking history information 600.

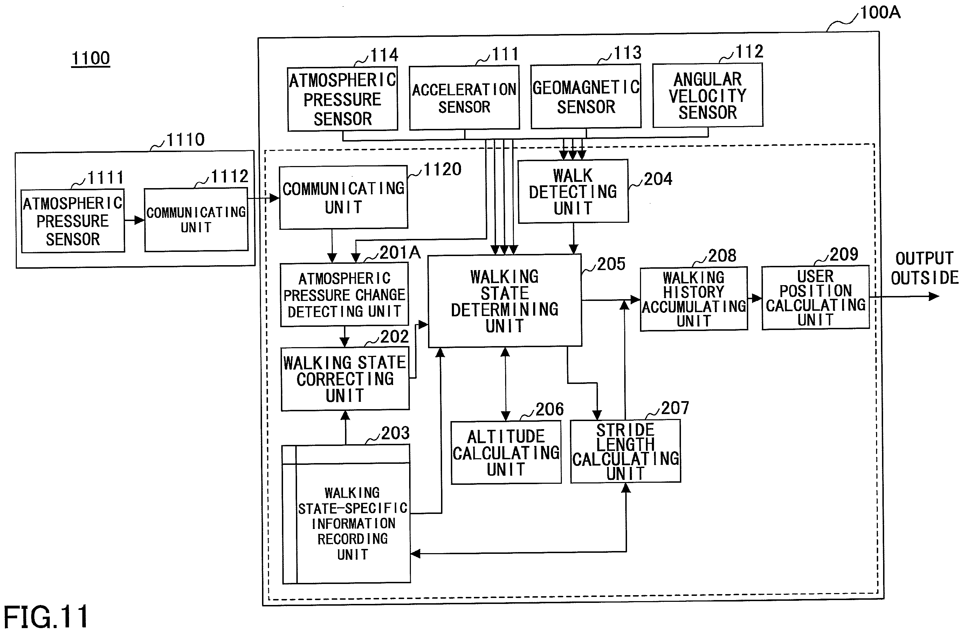

In the following, second, third, and fourth embodiments will be described with reference to FIGS. 11 to 16B.

FIG. 11 illustrates functional blocks of a navigation system 1100 according to the second embodiment. In the second embodiment, an information processing apparatus 100A receives atmospheric pressure information from an external communication apparatus 1110 and uses the atmospheric pressure information in determining the walking state of the user. The navigation system 1100 illustrated in FIG. 11 includes, for example, the external communication apparatus 1110 installed in a predetermined structure, and the information processing apparatus 100A having a function of receiving the atmospheric pressure information from the external communication apparatus 1110 and using the received atmospheric pressure information in determining a walking state. The information processing apparatus 100A includes the same configurations and functions as the information processing apparatus 100 according to the first embodiment described above, and descriptions of the same configurations and functions as those of the information processing apparatus 100 according to the first embodiment are omitted.

The external communication apparatus 1110 includes an atmospheric pressure sensor 1111 and a communicating unit 1112. The external communication apparatus 1110 acquires atmospheric pressure information of a site where the external communication apparatus 1110 is installed and transmits the information to the information processing apparatus 100A via the communicating unit 1112. On the other hand, the information processing apparatus 100A includes a communicating unit 1120 that receives atmospheric pressure information from the external communication apparatus 1110. The communicating unit 1112 and the communicating unit 1120 described above can communicate based on a communication protocol such as Bluetooth (registered trademark), for example; however, the communication protocol is not limited a such, and infrared data communication such as IrDa or wireless communication such as IEEE.802.11x may also be used.

The communicating unit 1120 of the information processing apparatus 100A transfers, to an atmospheric pressure change detecting unit 201A, the atmospheric pressure information of the site where the external communication apparatus 1110 is installed, received from the external communication apparatus 1110. When the atmospheric pressure information from the external communication apparatus 1110 and the atmospheric pressure information from the atmospheric pressure sensor 114 in the information processing apparatus 100A are both available as usable atmospheric pressure information, the atmospheric pressure change detecting unit 201A preferentially uses the atmospheric pressure information from the external communication apparatus 1110 to determine whether a "rapid atmospheric pressure change" has occurred. When a communication link with the external communication apparatus 1110 cannot be established or the atmospheric pressure information cannot be received from the external communication apparatus 1110 for a predetermined time, the atmospheric pressure change detecting unit 201A detects a "rapid atmospheric pressure change" by using the atmospheric pressure information acquired by the atmospheric pressure sensor 114 inside the information processing apparatus 100A that is an example of an atmospheric pressure detecting unit.

Except for the points described above, the atmospheric pressure change detecting unit 201A according to the second embodiment has the same configuration and functions as those of the atmospheric pressure change detecting unit 201 according to the first embodiment. When the atmospheric pressure change detecting unit 201A detects a rapid atmospheric pressure change, the atmospheric pressure change detecting unit 201A transfers the detection result to the walking state correcting unit 202. As a result, the information processing apparatus 100A executes the processes described above with reference to FIGS. 5 to 10A and 10B, similar to the case of the first embodiment described above, and determines the walking state to be recorded in the walking history information 600, by a method different from the original determination method (that is, by using the past data or the latest data, for example), similar to the first embodiment described above. Note that the atmospheric pressure information from the external communication apparatus 1110 can be smoothed by using an appropriate band-pass filter at the communicating unit 1120.

Note that in the navigation system 1100 according to the second embodiment illustrated in FIG. 11, when the external communication apparatus 1110 is communicatively connected to the information processing apparatus 100A, in a case where a rapid atmospheric pressure change is detected based on atmospheric pressure information from the external communication apparatus 1110, the navigation system 1100 determines the "walking state" to be recorded in the walking history information 600 by a method different from the original determination method (that is, for example, by using the past data or the latest data), similar to the first embodiment. When there is no external communication apparatus 1110 communicatively connected to the information processing apparatus 100A, in a case where a rapid atmospheric pressure change is detected based on atmospheric pressure information from the atmospheric pressure sensor 114, the navigation system 1100 can determine the "walking state" to be recorded in the walking history information 600 by a method different from the original determination method (that is, for example, by using the past data or the latest data), similar to the first embodiment.

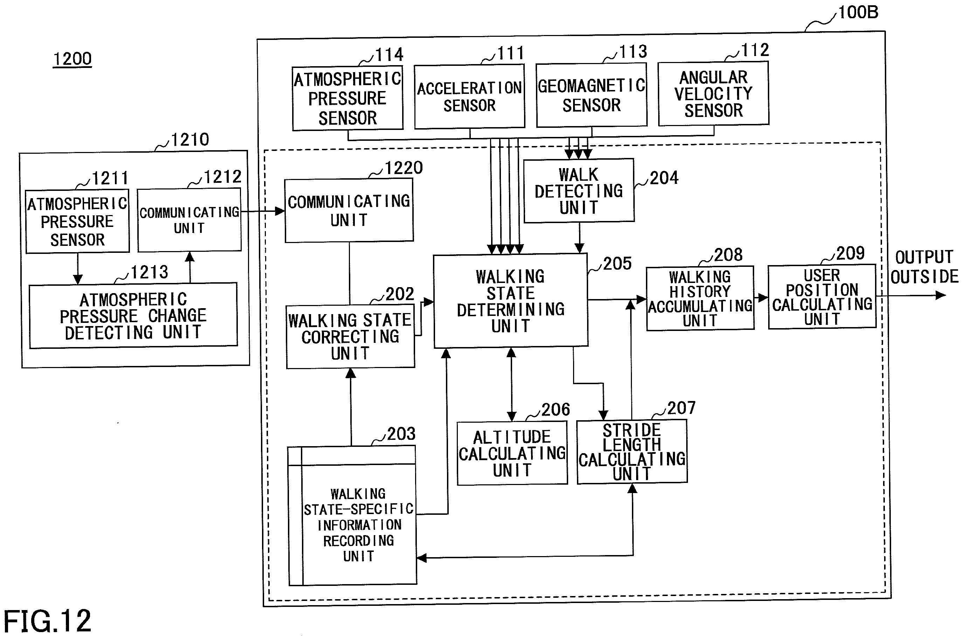

FIG. 12 illustrates functional blocks of a navigation system 1200 according to the third embodiment. In the third embodiment, an external communication apparatus 1210 includes an atmospheric pressure change detecting unit 1213. When the atmospheric pressure change detecting unit 1213 detects a "rapid atmospheric pressure change" based on the atmospheric pressure information at the site where the external communication apparatus 1210 is installed, acquired by an atmospheric pressure sensor 1211, the atmospheric pressure change detecting unit 1213 transmits the detection result from a communicating unit 1212 to a communicating unit 1220 in an information processing apparatus 100B. When the information processing apparatus 100B receives the detection result of the rapid atmospheric pressure change from the external communication apparatus 1210, the information processing apparatus 100B determines the "walking state" to be recorded in the walking history information 600 stored in the walking history accumulating unit 208, by a method different from the original determination method (that is, by using the past data or the latest data, for example), similar to the first embodiment. The information processing apparatus 100B includes the same configuration and functions as those of the information processing apparatus 100 according to the above-described first embodiment, and descriptions of the same configuration and functions as those of the information processing apparatus 100 according to the first embodiment are omitted. The communicating unit 1212 and the communicating unit 1220 described above can also communicate based on a communication protocol such as Bluetooth (registered trademark), similar to the above-described second embodiment; however, the communication protocol is not limited as such, and infrared data communication such as IrDa or wireless communication such as IEEE.802.11x may also be used.

The communicating unit 1220 of the information processing apparatus 100B immediately transfers the detection result of the rapid atmospheric pressure change received from the external communication apparatus 1210 to the walking state correcting unit 202. Subsequently, the information processing apparatus 100B executes the processes described above with reference to FIGS. 5 to 10A and 10B, similar to the case of the first embodiment described above, and determines the walking state to be recorded in the walking history information 600, by a method different from the original determination method (that is, by using the past data or the latest data, for example), similar to the first embodiment described above. Note that the detection result of the rapid atmospheric pressure change from the external communication apparatus 1210 can be smoothed by using an appropriate band-pass filter at the communicating unit 1220, similar to the second embodiment.

According to the navigation system 1200 according to the third embodiment illustrated in FIG. 12, the "walking state" to be recorded in the walking history information 600 based on the detection result of a rapid atmospheric pressure change is acquired from the external communication apparatus 1210, and therefore the processing load of the information processing apparatus 100B can be reduced. Note that in the navigation system 1200, when a communication link cannot be established with the external communication apparatus 1210, or when the detection result of a "rapid atmospheric pressure change" cannot be received from the external communication apparatus 1210 for a predetermined time, the information processing apparatus 100B does not include an atmospheric pressure change detecting unit, and therefore it is not possible to detect a rapid atmospheric pressure change. Therefore, in this case, the information processing apparatus 100B terminates the process of determining the walking state.

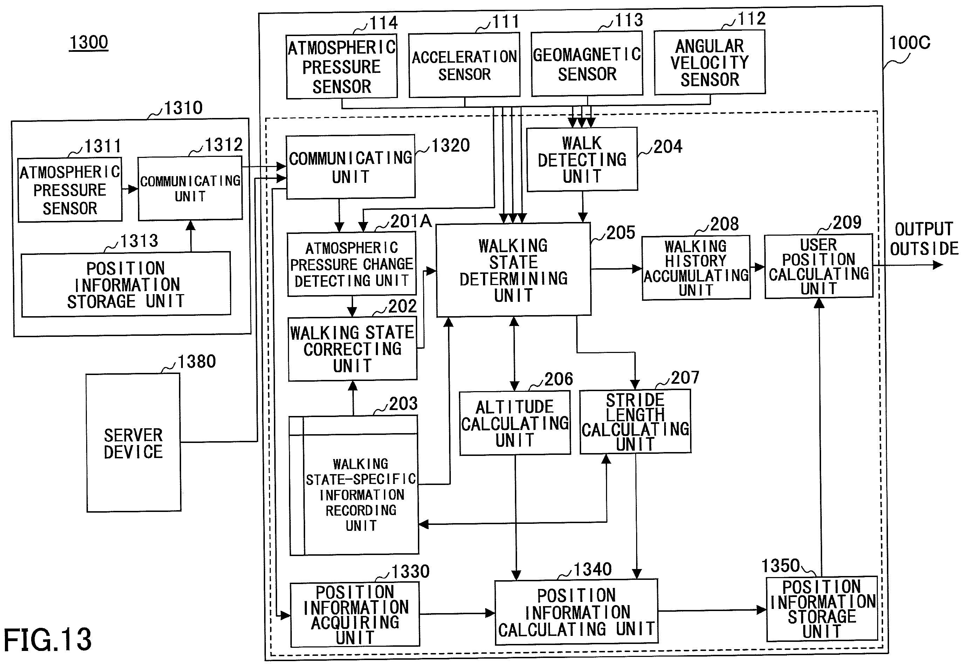

FIG. 13 illustrates functional blocks of a navigation system 1300 according to the fourth embodiment. In the fourth embodiment, by using the position information acquired from an external communication apparatus 1310, it is determined whether to use the atmospheric pressure information from the external communication apparatus 1310. The navigation system 1300 illustrated in FIG. 13 includes the external communication apparatus 1310 and an information processing apparatus 100C that has a function of receiving atmospheric pressure information from the external communication apparatus 1310 and using the received atmospheric pressure information in determining the walking state of the user. The information processing apparatus 100C includes the same configuration and functions as those of the information processing apparatus 100 according to the above-described first embodiment, and descriptions of the same configuration and functions as those of the information processing apparatus 100 according to the first embodiment are omitted.

The external communication apparatus 1310 includes an atmospheric pressure sensor 1311, a position information storage unit 1313, and a communicating unit 1312. As the position information stored in the position information storage unit 1313, information indicating the latitude, the longitude, and the altitude of the installation position of the external communication apparatus 1310 may be included (that is, latitude information, longitude information, and altitude information). Furthermore, instead of the position information, an identification value for uniquely identifying the external communication apparatus 1310 may be used. In the case where the identification value is used instead of the position information, the information processing apparatus 100C can acquire the position information corresponding to the identification value by sending a query to a server device 1380 installed outside. Alternatively, by storing the information indicating the association relationship between the position information and the identification value in a storage device or a storage area in the information processing apparatus 100C, the information processing apparatus 100C may obtain the position information corresponding to the identification value received from the external communication apparatus 1310. Furthermore, another information processing apparatus may be used instead of the external communication apparatus 1310, and the information processing apparatus 100C may acquire information indicating the association relationship between the position information and the identification value from the other information processing apparatus, or the information processing apparatus 100C may refer to the association relationship between the position information and the identification value included in the other information processing apparatus and obtain the corresponding position information. Furthermore, the position information may further include information indicating "whether the external communication apparatus 1310 is installed at a location where the surroundings are flat".

The external communication apparatus 1310 acquires atmospheric pressure information of a site where the external communication apparatus 1310 is installed, from the atmospheric pressure sensor 1311, and transmits, via the communicating unit 1312, the atmospheric pressure information and the position information or the identification value from the position information storage unit 1313. The information processing apparatus 100C includes a communicating unit 1320 that receives atmospheric pressure information and position information or an identification value from the external communication apparatus 1310. The communicating unit 1320 transfers the atmospheric pressure information received from the external communication apparatus 1310, to the atmospheric pressure change detecting unit 201A. The atmospheric pressure change detecting unit 201A has the same configuration as the atmospheric pressure change detecting unit 201A in the information processing apparatus 100A according to the second embodiment. When the information processing apparatus 100C can establish a communication link with the external communication apparatus 1310 and can receive the atmospheric pressure information from the external communication apparatus 1310, and the external communication apparatus 1310 is installed at a location where the surroundings are flat, the atmospheric pressure change detecting unit 201A detects a rapid atmospheric pressure change by using the atmospheric pressure information from the external communication apparatus 1310. On the other hand, when the information processing apparatus 100C cannot establish a communication link with the external communication apparatus 1310 or cannot receive the atmospheric pressure information from the external communication apparatus 1310 for a predetermined time, or when the external communication apparatus 1310 is not installed at a location where the surroundings are flat, the atmospheric pressure change detecting unit 201A detects a rapid atmospheric pressure change by using the atmospheric pressure information acquired by the atmospheric pressure sensor 114 inside the information processing apparatus 100C.
The communicating unit 1312 and the communicating unit 1320 described above can communicate based on a communication protocol such as Bluetooth (registered trademark), for example; however, the communication protocol is not limited as such, and infrared data communication such as IrDa or wireless communication such as IEEE.802.11x may also be used.

Furthermore, the communicating unit 1320 transfers the received position information or the identification value to a position information acquiring unit 1330. The position information acquiring unit 1330 transfers the transferred position information of the external communication apparatus 1310 to a position information calculating unit 1340 or acquires position information corresponding to the identification value and transfers the position information to the position information calculating unit 1340. The position information calculating unit 1340 holds the position information until the transferred position information is updated, for example, by receiving the position information from the external communication apparatus again. The position information calculating unit 1340 calculates the position of the information processing apparatus 100C by using the held position information and information from the altitude calculating unit 206 and the stride length calculating unit 207. The calculated position information indicating the present position of the information processing apparatus 100C is transferred from the position information calculating unit 1340 to a position information storage unit 1350 and is further transferred to the user position calculating unit 209.

Furthermore, in a case where the information processing apparatus 100C according to the fourth embodiment determines that the external communication apparatus 1310 is installed at a location where the surroundings are flat based on the position information received from the external communication apparatus 1310, and the walking state determining unit 205 determines that the walking state is an ascending/descending state, the walking state correcting unit 202 determines that the determination is an error. As a result, the walking state correcting unit 202 corrects the determination of the walking state to flat walking and records flat walking in the walking history information 600. This is because, as described above, the external communication apparatus 1310 to which the information processing apparatus 100C is communicably connected, is installed at a location where the surroundings are flat, and therefore it is erroneous to determine an ascending/descending state as the walking state of the user of the information processing apparatus 100C that is communicably connected to the external communication apparatus 1310.

Furthermore, when there are a plurality of the external communication apparatuses 1310 (including other information processing apparatuses) within a communicable distance, the order of priority for determining from which external communication apparatus 1310, from among the plurality of the external communication apparatus 1310, information is to be used, may be established based on the accuracy of the position information of each external communication apparatus 1310, the installation state of the external communication apparatus (fixed or moving, etc.), and information indicating the proximity state with the information processing apparatus 100C (the same building, the same floor, or the same room, etc.).

Similar to the first embodiment, when the atmospheric pressure change detecting unit 201A of the information processing apparatus 100C detects a rapid atmospheric pressure change, the atmospheric pressure change detecting unit 201A transfers the detection result to the walking state correcting unit 202. Subsequently, the information processing apparatus 100C executes the processes described above with reference to FIG. 5 to FIG. 10A and FIG. 10B, similar to the case of the first embodiment described above, and determines the "walking state" to be recorded in the walking history information 600, by a method different from the original determination method (that is, by using the past data or the latest data, for example). However, in the case of the fourth embodiment, as described above, when it is determined that the external communication apparatus 1310 is installed at a location where the surroundings are flat based on the position information received from the external communication apparatus 1310, and the walking state determining unit 205 determines that the walking state is an ascending/descending state (or further in a case where the walking state correcting unit 202 once determines that the walking state is an ascending/descending state by using the past data or the latest data according to the detection of a rapid atmospheric pressure change), the walking state correcting unit 202 determines that the determination is an error. As a result, the walking state correcting unit 202 corrects the determination of the walking state to "flat walking" and records flat walking in the walking history information 600. Note that the atmospheric pressure information from the external communication apparatus 1310 can be smoothed by the communicating unit 1320 by using an appropriate band-pass filter.

When the information processing apparatus 100C is not communicably connected to the external communication apparatus 1310 (that is, a communication link is not established), the walking history accumulating unit 208 and the user position calculating unit 209 of the information processing apparatus 100C cannot acquire position information or atmospheric pressure information from the communicating unit 1320. In such a case, the information processing apparatus 100C determines the walking state of the user in the same manner as in the first embodiment, calculates the present position of the user based on the determination result, and outputs the present position to the outside. Conversely, when the information processing apparatus 100C is communicably connected to the external communication apparatus 1310 (that is, a communication link is established), the user position calculating unit 209 acquires the latest position information from the position information storage unit 1350 and outputs the position information to the outside as the position information of the information processing apparatus 100C. In the navigation system 1300 according to the fourth embodiment, for example, by using a position information notification device such as i-Beacon as the external communication apparatus 1310, the information processing apparatus 100C can correct the determination result of the position, which is obtained by Pedestrian Dead Reckoning, according to position information from the external communication apparatus 1310, enabling more accurate navigation.

According to the navigation system 1300 illustrated in FIG. 13, when the external communication apparatus 1310 is communicably connected to the information processing apparatus 100C, and a rapid atmospheric pressure change is detected based on atmospheric pressure information from the external communication apparatus 1310, the "walking state" to be recorded in the walking history information 600 is determined by a method different from the original determination method (that is, for example, by using the past data or the latest data), similar to the case of the first embodiment. However, in the case of the fourth embodiment, as described above, when it is determined that the external communication apparatus 1310 is installed at a location where the surroundings are flat based on the position information received from the external communication apparatus 1310, and the walking state determining unit 205 determines that the walking state is an ascending/descending state (or when the walking state correcting unit 202 determines that the walking state is an ascending/descending state by using the past data or the latest data upon detecting a rapid atmospheric pressure change), the walking state correcting unit 202 determines that the determination is an error. As a result, the position information calculating unit 1340 corrects the determination of the walking state to flat walking and records flat walking in the walking history information 600. Conversely, when the external communication apparatus 1310 is not provided, and a rapid atmospheric pressure change is detected based on atmospheric pressure information from the atmospheric pressure sensor 114, the information processing apparatus 100C determines the "walking state" to be recorded by a method different from the original determination method (that is, for example, by using the past data or the latest data), similar to the case of the first embodiment. Furthermore, it is possible to correct errors that that may be included in the position information calculated by the information processing apparatus 100C by Pedestrian Dead Reckoning, by using the position information from the external communication apparatus 1310, enabling more accurate navigation.

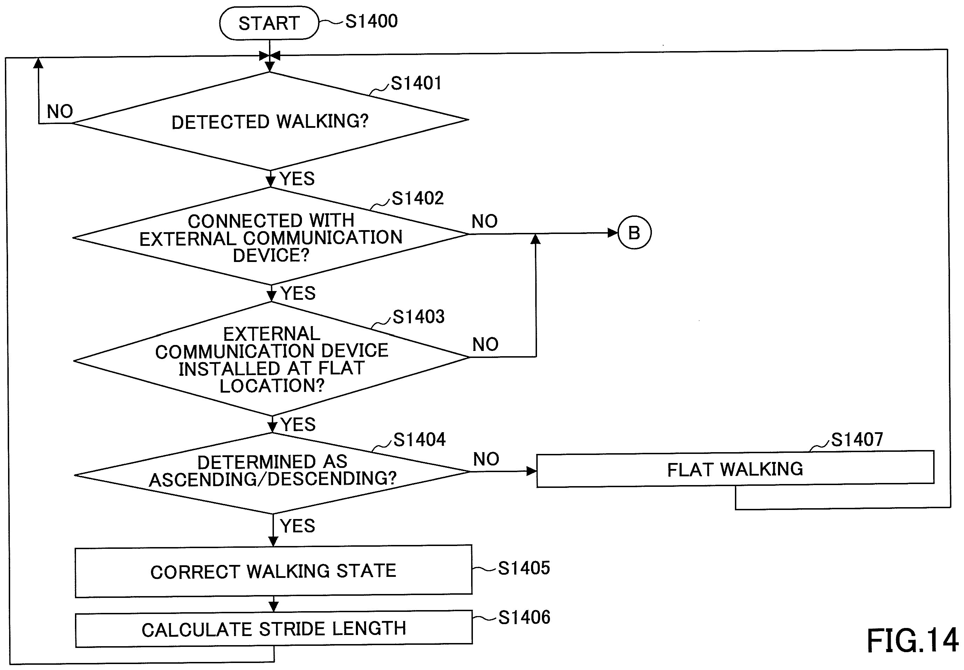

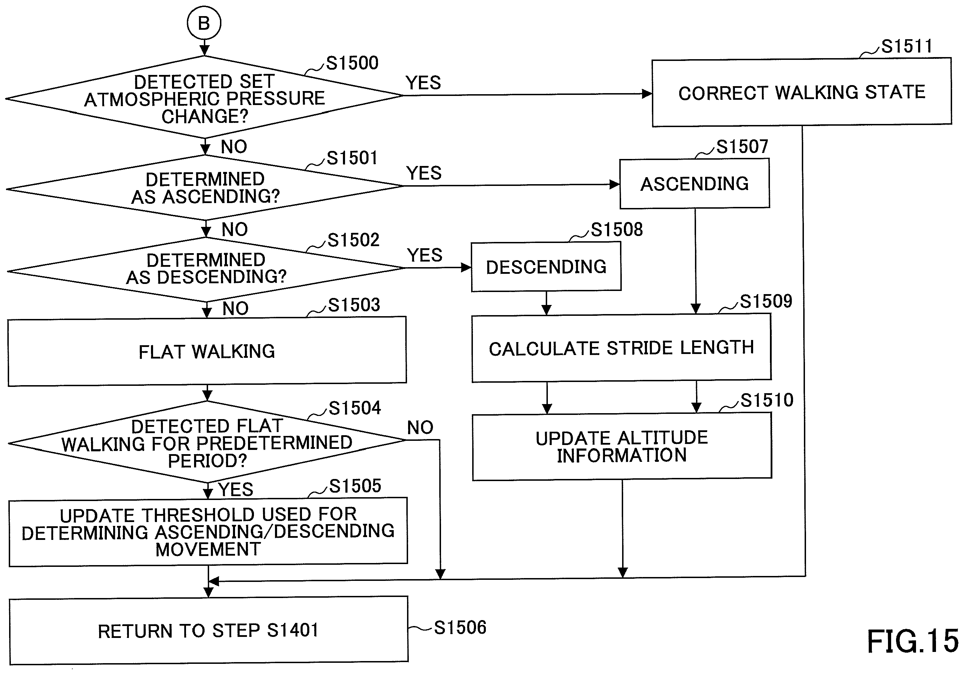

Next, with reference to FIGS. 14 and 15, details of the processes of the information processing apparatus 100C in the navigation system 1300 according to the fourth embodiment described above with reference to FIG. 13, will be described. Note that the information processing apparatus 100A in the navigation system 1100 according to the second embodiment is different in that the atmospheric pressure sensor 1111 of the external communication apparatus 1110 is used for obtaining the atmospheric pressure information to be used in some cases; otherwise, the processes are similar to those of the first embodiment, and therefore overlapping descriptions will be omitted. Furthermore, the information processing apparatus 100B in the navigation system 1200 according to the third embodiment is different in that the atmospheric pressure change detecting unit 1213 of the external communication apparatus 1210 detects a rapid atmospheric pressure change; otherwise, the processes are similar to those of the first embodiment, and therefore overlapping descriptions will be omitted.

When the process in FIG. 14 is started by, for example, a user's operation at step S1400, in step S1401, the walk detecting unit 204 detects the user's walking by using the acceleration information detected by the acceleration sensor 111. Then, the walk detecting unit 204 determines whether the detected walking has continued for the predetermined criterion. When the detected walking has not continued for the predetermined criterion (NO), in order to acquire an initial value of the atmospheric pressure information, the walking state determining unit 205 stores atmospheric pressure data of the period during which the walking has continued, and returns to step S1401.

In step S1401, when the walk detecting unit 204 determines that the detected walking has continued for the predetermined criterion (YES), next, in step S1402, the communicating unit 1320 determines whether the information processing apparatus 100C is communicably connected to the external communication apparatus 1310. When the information processing apparatus 100C is not communicably connected to the external communication apparatus 1310 (NO in step S1402), the processes from step S1500 an onward in FIG. 15 are executed. Note that the predetermined criterion for determining whether the walking has continued, can be set by using the walking time, or the number of steps, etc., similar to the first embodiment. For example, it can be determined that the detected walking has continued for the predetermined criterion, when the detected walking has continued for five steps when the criterion is the number of steps, or when the detected walking has continued for 2.5 seconds when the criterion is the walking time. The predetermined criterion for determining whether the walking has continued, can be appropriately set according to the walking characteristics, etc., of the user.

In step S1402, when it is determined that the information processing apparatus 100C is communicably connected to the external communication apparatus 1310 (YES), in step S1403, the walking state determining unit 205 uses the position information of the external communication apparatus 1310 to determine whether the communicably connected external communication apparatus 1310 is installed at a location where the surroundings are flat. When the communicably connected external communication apparatus 1310 is installed at a location where the surroundings are not flat (NO in step S1403), the processes from step S1500 and onward in FIG. 15 are executed. Conversely, when the communicably connected external communication apparatus 1310 is installed at a location where the surroundings are flat (YES in step S1403), step S1404 is executed.

In step S1404, the walking state determining unit 205 performs the same processes as those of the steps S500 to S503, S507, S508, and S511 described above with reference to FIG. 5 (however, the process corresponding to step S500 is performed by using the atmospheric pressure information from the external communication apparatus 1310), thereby determining the walking state of the user. As described above, when the external communication apparatus 1310 communicably connected to the information processing apparatus 100C is installed at a location where the surroundings are flat, and the walking state determining unit 205 determines the walking state is an ascending/descending state, it is determined that the determination is an error. Therefore, when the walking state determining unit 205 or the walking state correcting unit 202 determines that the walking state is an ascending/descending state (YES in step S1404), the walking state correcting unit 202 determines that the determination is an error and corrects the determination to flat walking. The walking state correcting unit 202 further records the corrected walking state (flat walking) in the walking history information 600 via the walking state determining unit 205. Furthermore, the stride length calculating unit 207 calculates the stride length according to "flat walking" and records the stride length in the walking history information 600 (step S1406) .

Conversely, when the walking state determining unit 205 determines that the walking state is flat walking in step S1404 (NO), the walking state determining unit 205 records "flat walking" as the walking state in the walking history information 600, and the stride length calculating unit 207 calculates the stride length according to the flat walking and records the calculated stride length in the walking history information 600 (step S1407). Subsequently, the processes from step S1401 and onward are repeated.

According to the fourth embodiment, it is possible to determine that the walking state is flat walking by using the position information received from the external communication apparatus 1310, and therefore in this case, the walking state can be determined more accurately.

FIG. 15 illustrates an example of a process of a case of determining the walking state when the information processing apparatus 100C cannot use the atmospheric pressure information from the external communication apparatus 1310, or when the information processing apparatus 100C can only communicate with the external communication apparatus 1310 that is installed at a location where the surroundings are not flat.

The process in FIG. 15 starts when the determination result in step S1402 or S1403 in FIG. 14 is NO. Note that the process of FIG. 15 is performed by using the atmospheric pressure information from the atmospheric pressure sensor 114 of the information processing apparatus 100C, by the process described above with reference to FIG. 5. In step S1500, it is determined whether the atmospheric pressure change detecting unit 201A has detected a "rapid atmospheric pressure change".

Also in the fourth embodiment, similar to the case of the first embodiment, the "rapid atmospheric pressure change" means a rapid atmospheric pressure change that does not occur during normal walking, such as a pressure change when a train arrives or departs in a subway station.

When a "rapid atmospheric pressure change" is not detected (NO in step S1500), the walking state determining unit 205 proceeds to step S1501 to determine whether the user's walking is an ascending motion, and when the user's walking is determined to be an ascending motion (YES), the walking state determining unit 205 proceeds to step S1507. In step S1507, the walking state determining unit 205 records an ascending motion (for example, "ascending staircase") as the walking state of the user in the walking history information 600.

Next, in step S1509, the stride length calculating unit 207 calculates the stride length by the method described above with reference to FIG. 4. Subsequently, in step S1510, the walking state determining unit 205 calculates altitude information by using the information indicating the level difference of stairs at the time of ascending/descending stored in the walking state-specific information recording unit 203 and the number of steps, and updates the walking history information 600 stored in the walking history accumulating unit 208 by using the calculated altitude information. Subsequently, step S1506 is executed, and furthermore, step S1401 in FIG. 14 is executed, whereby the process described above with reference to FIG. 14 is repeated.

In the calculation of the "stride length" described above, for example, in the calculation of the walking speed at the time of ascending/descending, the stride length calculating unit 207 calculates the walking speed in the horizontal direction at the time of ascending/descending by multiplying the walking speed calculated by using the acceleration in the vertical direction by a coefficient considering the inclination and the level difference of stairs, etc., and further calculates the stride length by using the calculated walking speed and the number of steps. Note that the coefficient considering the inclination and the level difference of stairs, etc., may be determined in advance based on a value of a general staircase or a slope (for example, a value of a standard of staircases), etc., and recorded in the walking state-specific information recording unit 203. Then, by reporting the stride length calculated by the stride length calculating unit 207 to the walking state determining unit 205, the walking state determining unit 205 records the stride length in the walking history information 600 stored in the walking history accumulating unit 208.

Conversely, when the user's walking is not determined to be an ascending motion in step S1501 (NO), the walking state determining unit 205 proceeds to step S1502 to determine whether the user's walking state is a descending motion. In step S1502, when the user's walking is determined to be a descending motion (YES), the walking state determining unit 205 proceeds to step S1508 and records the walking state of the user as a descending motion (for example, "descending staircase") in the walking history information 600.

Next, in step S1509, the stride length calculating unit 207 calculates the stride length by the method described above with reference to FIG. 4. Subsequently, in step S1510, the walking state determining unit 205 calculates the altitude information by using the information indicating the level difference of stairs stored in the walking state-specific information recording unit 203 and the number of steps, and updates the walking history information 600 stored in walking history accumulating unit 208 by using the calculated altitude information. Subsequently, step S1506 is executed, and furthermore, step S1401 in FIG. 14 is executed, whereby the process described above with reference to FIG. 14 is repeated.

Conversely, when the user's walking is not determined to be a descending motion in step S1502 (NO), in step S1503, the walking state determining unit 205 determines the walking state of the user as flat walking, and records the walking state as "flat walking" in the walking history information 600. Next, the stride length calculating unit 207 calculates the stride length according to the flat walking and records the stride length in the walking history information 600. Furthermore, the walking state determining unit 205 proceeds to step S1504 to determine whether flat walking has been continuously detected for a predetermined time. When flat walking is not continuously detected for a predetermined time (NO in step S1504), step S1506 is executed, and step S1401 in FIG. 14 is further executed, thereby repeating the process described above with reference to FIG. 14.

Furthermore, when flat walking is continuously detected for a predetermined time in step S1504 (YES), the walking state determining unit 205 proceeds to step S1505. In step S1505, the walking state determining unit 205 uses a variance value of the acceleration in the vertical direction at the time of flat walking to update a threshold that is referred to when determining whether the walking state of the user is ascending/descending a staircase, that is a threshold stored in the walking state-specific information recording unit 203. As a result, the accuracy of determining whether the walking state is ascending/descending a staircase is improved. Subsequently, the walking state determining unit 205 proceeds to step S1506, further proceeds to step S1401 in FIG. 14, and repeats the process described above with reference to FIG. 14.

Conversely, when a "rapid atmospheric pressure change" is detected in step S1500 (YES), the walking state correcting unit 202 determines that the walking state will be erroneously determined due to a factor other than the user's movement such as ascending/descending a staircase (for example, a factor such as a rapid atmospheric pressure change accompanying the passage of a train in a station). Therefore, the walking state correcting unit 202 proceeds to step S1511 to determine the "walking state" in the walking history information 600 stored in the walking history accumulating unit 208, by a method different from the original determination method. The determination of the walking method by a method different from the original determination method is performed by the walking state correcting unit 202, for example, by using the "walking state" determined before the atmospheric pressure change is detected, as the "walking state" to be recorded after the "rapid atmospheric pressure change" is detected, in the walking history information 600 stored in the walking history accumulating unit 208, similar to the case of the first embodiment.

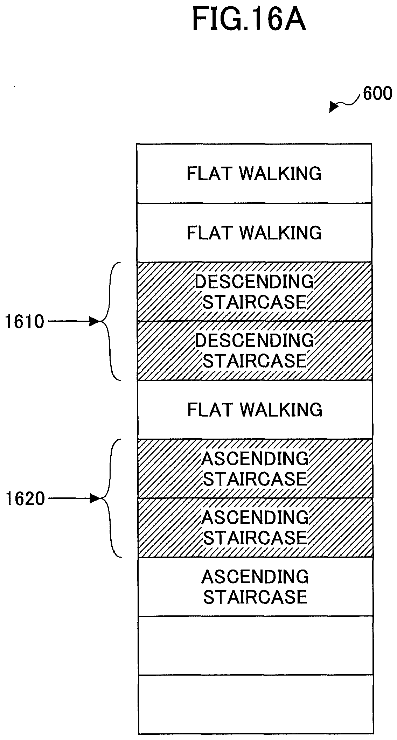

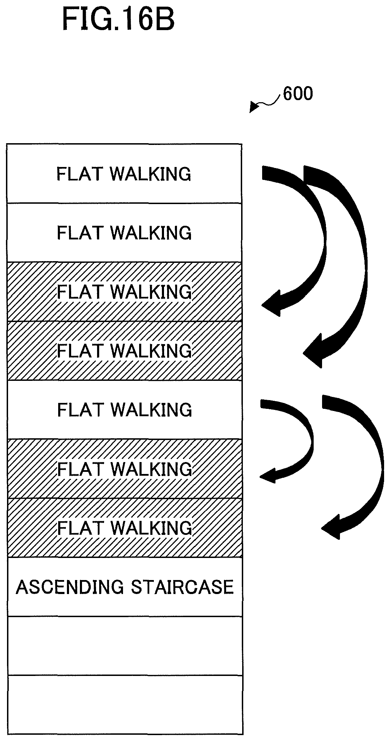

FIGS. 16A and 16B are diagrams for describing an example of determination by a method different from the original determination method of determining the "walking state" in the walking history information 600 according to the fourth embodiment. In FIGS. 16A and 16B, the initially determined "walking state" and the "walking state" to be recorded are the same as in the examples of FIGS. 10A and 10B.

In FIGS. 16A and 16B, a walking state 1610 is a walking state determined by the walking state determining unit 205 when a train enters a platform, and a walking state 1620 is a walking state determined by the walking state determining unit 205 when the train passes the platform. During these states, the atmospheric pressure sensor 1311 or the atmospheric pressure sensor 114 detects a "rapid atmospheric pressure change" as illustrated in FIG. 8 or 9. As a result, the walking state correcting unit 202 of the information processing apparatus 100C determines the "walking state" to be recorded in the walking history information 600, by a method different from the original determining method (that is, for example, by using the past data or the latest data), similar to the first embodiment. However, in the case of the fourth embodiment, as described above, when the external communication apparatus 1310 is determined to be installed at a location where the surroundings are flat based on position information received from the external communication apparatus 1310, and the walking state determining unit 205 determines that the walking state is an ascending/descending state, the walking state correcting unit 202 determines that the determination is an error. As a result, the position information calculating unit 1340 corrects the determination of the walking state to flat walking, and records flat walking in the walking history information 600. The stride length calculating unit 207 of the information processing apparatus 100C calculates the "stride length" to be recorded in the walking history information 600 according to the determined walking state.

According to the fourth embodiment, when the determination result of step S1403 in FIG. 14 is YES, it can be determined that the walking state is flat walking, and therefore the determination can be made more reliably.

As described above, according to the embodiments of the present invention, it is possible to provide an information processing system, an information processing apparatus, an information processing method, a program, and a recording medium capable of accurately determining a moving state of a movable body.

The information processing system, the information processing apparatus, the information processing method, the program, and the recording medium are described by embodiments; however, the present invention is not limited to the specific embodiments described in the detailed description, and variations and modifications may be made without departing from the spirit and scope of the present invention.

The present international patent application claims the benefit of priority of Japanese Patent Application No. 2015-247206, filed on December 18, 2015 and Japanese Patent Application No. 2016-112425, filed on June 6, 2016, the entire contents of which Japanese Patent Application No. 2015-247206 and Japanese Patent Application No. 2016-112425 are hereby incorporated herein by reference.

### [Reference Signs List]

100, 100A, 100B, 100C information processing apparatus
101 microphone
102 speaker
103 first communicating unit
104 second communicating unit
105 position information receiving unit
106 display unit
107 input unit
108 CPU
109 RAM
110 ROM
111 acceleration sensor
112 angular velocity sensor
113 geomagnetic sensor
114 atmospheric pressure sensor
201, 201A atmospheric pressure change detecting unit
202 walking state correcting unit
203 walking state-specific information recording unit
204 walk detecting unit
205 walking state determining unit
206 altitude calculating unit
207 stride length calculating unit
208 walking history accumulating unit
209 user position calculating unit
1100, 1200, 1300 navigation system

### [Citation List]

### [Patent Literature]

[PTL 1]
   Japanese Unexamined Patent Application Publication No. 2015-29884

### [Non Patent Literature]

[NPL 1]
   Kourogi, Masakatsu., Takeshi Kurata, "A method of personal positioning based on walking motion analysis using inertial sensor group and wearable camera", IEICE technical report, PRMU2004, volume 103, 2004, pp.25-30.

## Claims

1. An information processing system comprising:
an atmospheric pressure detector configured to detect atmospheric pressure surrounding an information processing apparatus;
a determiner configured to determine a movement state of a movable body by using at least a detection result obtained by the atmospheric pressure detector; and
a corrector configured to determine the movement state of the movable body by a method different from a method used by the determiner, in response to detecting a predetermined change in the atmospheric pressure detected by the atmospheric pressure detector.

2. The information processing system according to claim 1, further comprising:
an external communication device configured to detect the atmospheric pressure, wherein
the information processing apparatus determines the movement state of the movable body by communicating with the external communication device.

3. The information processing system according to claim 2, wherein
the information processing apparatus detects the atmospheric pressure by using the atmospheric pressure detector, in response to determining that atmospheric pressure information from the external communication device cannot be used.

4. The information processing system according to any one of claims 1 to 3, wherein
the predetermined change in the atmospheric pressure is a change that is more rapid than a change caused by a movement of the movable body, and
the corrector determines the movement state of the movable body by the method different from the method used by the determiner, in response to detecting the predetermined change in the atmospheric pressure.

5. An information processing apparatus for determining a movement state of a movable body including at least ascending/descending of the movable body, the information processing apparatus comprising:
an atmospheric pressure detector configured to detect atmospheric pressure surrounding the information processing apparatus;
a determiner configured to determine the movement state of the movable body by using at least a detection result obtained by the atmospheric pressure detector; and
a corrector configured to determine the movement state of the movable body by a method different from a method used by the determiner, in response to detecting a predetermined change in the atmospheric pressure detected by the atmospheric pressure detector.

6. The information processing apparatus according to claim 5, wherein
the movement state indicates a walking state of a pedestrian, and wherein
the information processing apparatus further comprises:
a stride length calculator configured to calculate a stride length according to the walking state.

7. The information processing apparatus according to claim 5 or 6, further comprising:
a recorder configured to record the movement state as movement history information in time series.

8. The information processing apparatus according to any one of claims 5 to 7, wherein the corrector retroactively corrects the movement state, which is determined by the determiner, in time series, in response to detecting the predetermined change in the atmospheric pressure.

9. The information processing apparatus according to any one of claims 5 to 8, wherein
the predetermined change in the atmospheric pressure is a change that is more rapid than a change caused by a movement of the movable body, and
the corrector determines the movement state of the movable body by the method different from the method used by the determiner, in response to detecting the predetermined change in the atmospheric pressure.

10. An information processing method performed by an information processing apparatus for determining a movement state of a movable body including at least ascending/descending of the movable body, the information processing method comprising:
an atmospheric pressure detecting step of detecting atmospheric pressure surrounding the information processing apparatus;
a determining step of determining the movement state of the movable body by using at least a detection result obtained at the atmospheric pressure detecting step; and
a correcting step of determining the movement state of the movable body by a method different from a method used at the determining step, in response to detecting a predetermined change in the atmospheric pressure detected at the atmospheric pressure detecting step.

11. The information processing method according to claim 10, wherein
the movement state indicates a walking state of a pedestrian, and wherein
the information processing method further comprises:
a step of calculating a stride length according to the walking state.

12. The information processing method according to claim 10 or 11, wherein the correcting step includes retroactively correcting the movement state, which is determined at the determining step, in time series, in response to detecting the predetermined change in the atmospheric pressure.

13. The information processing method according to any one of claims 10 to 12, wherein
the predetermined change in the atmospheric pressure is a change that is more rapid than a change caused by a movement of the movable body, and
at the correcting step, the movement state of the movable body is determined by the method different from the method used at the determining step, in response to detecting the predetermined change in the atmospheric pressure.

14. A computer-executable program for causing a computer to function as the information processing apparatus according to any one of claims 5 to 9.

15. A computer-readable recording medium storing the program according to claim 14.
